# FASCICULE DE BREVET EUROPEEN

(11) **EP 2 129 598 B1**
(45) Date de publication et mention de la délivrance du brevet: **18.04.2012**
(21) Numéro de dépôt: 07870388.1
(22) Date de dépôt: 11.12.2007
(51) Int. Cl.: B65D 83/14, A61M 15/00

(54) **POCHE DE VOLUME VARIABLE, DISPOSITIF APTE A DELIVRER DES FLUIDES COMPRENANT LADITE POCHE ET PROCEDE DE REMPLISSAGE DUDIT DISPOSITIF**
TASCHE VON VERÄNDERLICHEM VOLUMEN, FLÜSSIGKEITSAUSGABEVORRICHTUNG MIT EINER SOLCHEN TASCHE UND VERFAHREN ZUR FÜLLUNG BESAGTER VORRICHTUNG
VARIABLE VOLUME POCKET, FLUID DISPENSING DEVICE COMPRISING SAID POCKET AND METHOD FOR FILLING SAID DEVICE

(30) Priorité: 14.12.2006 US 610842; 14.02.2007 WO PCT/IB2007/000342; 14.08.2007 US 955748 P; 14.08.2007 FR 0705855
(43) Date de publication de la demande: 09.12.2009
(73) Titulaire: POWER CONTAINER CORP., 08873 Somerset, New Jersey (US)
(72) Inventeur: NIMMO, Chris, Amersham Buckinghamshire HP6 5HD (GB); BERTAUD, Olivier, 92100 Boulogne (FR)
(74) Mandataire: Cabinet Plasseraud
(86) Numéro de dépôt international: PCT/FR2007/052475
(87) Numéro de publication internationale: WO 2008/078037

(56) Documents cités:
- WO-A-2006/087462
- US-A- 4 964 540
- US-A1- 2004 226 964

## Description

L'invention a pour objet une poche de volume variable destinée à être introduite par le goulot d'un récipient externe sous pression avant son remplissage par un fluide. L'invention porte également sur ledit dispositif apte à délivrer des fluides qui comprend la poche conforme à l'invention ainsi qu'un procédé de remplissage dudit dispositif.

Dans de nombreux domaines industriels, et notamment dans ceux des produits détergents, des médicaments et des produits cosmétiques, des fluides, et principalement des liquides contenant des agents nettoyants, désinfectants, des substances médicamenteuses ou cosmétiques, doivent être délivrés notamment par pulvérisation, éventuellement sous pression pour les besoins de leur utilisation.

Il en est ainsi à titre d'exemple de l'eau de mer isotonique qui a des applications notamment pour le lavage des fosses nasales.

Il existe déjà des dispositifs du genre en question dont les caractéristiques sont décrites dans les brevets américains n° 4387833,4423829, 5927551 et 4964540 ou bien dans les documents US-A-2004 226 964 et WO-A-2006 087 462.

Dans le cas de ces dispositifs, qui peuvent fonctionner dans toutes les positions et même en position retournée, le fluide est délivré non pas sous l'action d'un gaz propulseur mais sous l'action d'une contrainte mécanique qui est exercée sur un récipient en forme de poche ou de sac de volume variable et de forme générale cylindrique à plis longitudinaux, rempli du fluide à délivrer.

Toujours dans le cas des dispositifs décrits dans les susdits brevets américains, la contrainte mécanique, sous l'action de laquelle le fluide contenu dans le récipient de volume variable est délivré sous pression, est exercée par un manchon cylindrique en matériau élastique, et notamment en un caoutchouc à propriétés élastiques particulières, qui entoure le récipient en forme de poche ou de sac et dont le diamètre est légèrement supérieur à celui du récipient à volume variable lorsque celui-ci est vide.

Le manchon en question est alors mis en place et le fluide à délivrer est introduit sous pression dans le récipient qui se dilate contre l'action contraire du manchon élastique dont la force de compression exercée sur le récipient augmente avec la dilatation de celui-ci par suite du remplissage par le fluide à délivrer.

Le récipient en forme de poche ou de sac est muni d'une commande permettant d'actionner un clapet en vue de la délivrance du fluide, l'ensemble étant disposé à l'intérieur d'un récipient ou réservoir classique du type de ceux utilisés dans l'industrie des aérosols, notamment en cosmétologie.

Ces dispositifs, qui sont très robustes, ont toujours donné entière satisfaction aux utilisateurs mais sont pénalises par leur prix de revient dû au coût du caoutchouc constitutif du manchon élastique entrant dans leur constitution.

Il existe d'autres dispositifs du genre en question dans lesquels un récipient de volume variable, destiné à être rempli du fluide à délivrer et équipé également d'une commande propre à actionner un clapet pour permettre de délivrer le fluide sous pression, est disposé à l'intérieur d'un récipient extérieur capable de résister à des pressions élevées, notamment supérieures à 20 bars ; ce récipient extérieur est rempli d'un gaz neutre sous pression, le récipient à volume variable vide étant en place, puis ce dernier est rempli du fluide à délivrer par l'introduction de ce dernier sous une pression suffisante pour vaincre la pression exercée sur le récipient par le gaz neutre remplissant le récipient extérieur, ce qui a pour conséquence d'accroître encore la pression du gaz neutre.

Dans le cas de ces dispositifs, la contrainte exercée sur le récipient intérieur à volume variable et grâce à laquelle le fluide sous pression peut être délivré est donc de nature pneumatique.

Ces dispositifs n'ont pas connu un succès comparable à ceux décrits plus haut, notamment en raison de leur fragilité en cas de choc ou de chute, notamment au niveau de la liaison entre le récipient de volume variable et la commande dont l'actionnement permet de délivrer le fluide sous pression.

D'autres dispositifs du genre en question ont également été décrits notamment dans DE-OS 2304538, US4969577, US5219006, US5505289, US5388716, US6345739, EP718213 et CH678614.

Ces dispositifs présentent certains inconvénients liés notamment à leur mode de remplissage ou à leur fragilité ou bien sont industriellement non réalisables.

Dans le brevet FR2882037, la Demanderesse a décrit un dispositif dépourvu des inconvénients des dispositifs de l'art antérieur et notamment beaucoup moins fragile que ces derniers. Cependant, lorsqu'il est soumis à des températures extrêmes, ledit dispositif peut présenter une certaine diminution de son étanchéité.

Or il est du mérite des inventeurs d'avoir trouvé que de façon surprenante et inattendue, il pouvait être remédié à ce défaut d'étanchéité dans des conditions extrêmes en mettant en oeuvre une poche de volume variable dont l'extrémité ouverte est stable dimensionnellement sur des plages de températures allant de -30 à 55°C, de préférence de -5 à 50°C et plus préférentiellement de 10 à 40°C.

En conséquence, l'invention porte sur une poche de volume variable apte à contenir des fluides et destinée à être introduite, vide, dans un récipient par le goulot de celui-ci, constituée d'un sac fermé à l'une de ses extrémités et ouverte à l'autre extrémité, l'extrémité ouverte étant stable dimensionnellement sur des plages de températures allant de -30 à 55°C, de préférence de -5 à 50°C et plus préférentiellement de 10 à 40°C.

Conformément à la présente invention, ladite extrémité ouverte est dite « stable dimensionnellement » dans la mesure où, dans une plage de températures de -30 à 55°C, de préférence de -5 à 50°C et plus préférentiellement de 10 à 40°C, le facteur de dilatation de ladite extrémité est divisé par 1,5, de préférence par 2 et plus préférentiellement encore par 3, par rapport à celui du reste de la poche.

Le facteur de dilatation est le rapport M/m, M étant la valeur maximale qui est atteinte par la plus grande dimension de ladite extrémité ouverte dans la plage de températures allant de -30 à 55°C, de préférence de -5 à 50°C et plus préférentiellement de 10 à 40°C, et m étant la valeur minimale qui est atteinte par la plus grande dimension de ladite extrémité ouverte dans la plage de températures allant de -30 à 55°C, de préférence de -5 à 50°C et plus préférentiellement de 10 à 40°C.

Cette stabilité dimensionnelle permet d'assurer l'étanchéité de dispositifs incorporant ladite poche de volume variable, quelque soit leur plage de températures d'utilisation et/ou de stockage pendant toute la durée de vie dudit dispositif.

Ainsi, les dispositifs sous pression incorporant ladite poche satisfont les exigences du règlement n° 842/2006 du parlement et du conseil européens et les exigences du Département américain des transports sur les transports et emballages (voir CFR 49, volume 2 : 49CFR173.306).

Ladite poche étant destinée à être adaptée sur tout type de récipient externe, elle peut être dotée de différents moyens permettant de la solidariser au récipient externe. Ainsi, selon un mode de réalisation particulier son extrémité libre est dotée d'un pas de vis, de telle sorte qu'elle sera rendue solidaire du récipient externe par vissage à l'intérieur du goulot dudit récipient externe. Dans ce mode de réalisation, au moins une partie, de préférence la totalité de l'extrémité libre dotée du pas de vis est stable dimensionnellement.

Selon un autre mode de réalisation, ladite extrémité ouverte est destinée à être sertie sur le récipient externe, directement ou par l'intermédiaire d'une coupelle comme décrit dans les demandes de brevet au nom de la Demanderesse FR0501511, FR0511614 et PCT/FR2006/000338, dont l'enseignement est incorporé par référence. Dans ce mode de réalisation, la paroi externe de l'extrémité ouverte comporte au moins une protubérance radiale destinée au sertissage.

Selon un autre mode de réalisation, le bord libre de l'extrémité ouverte présente au moins une bague radiale destinée à une soudure sur le récipient externe.

Compte tenu du fait que la poche est de volume variable, le matériau constitutif de celle-ci doit autoriser cette variabilité de volume.

Ainsi, la poche de volume variable est avantageusement en polyéthylènetéréphtalate ou en polyéthylène naphtalate ou, en tout autre matériau synthétique approprié offrant des propriétés analogues.

En particulier, la poche de volume variable peut être réalisée à l'aide de laminés dont l'une au moins des couches constitutives confère au laminé la résistance mécanique suffisante, une autre couche pouvant conférer des propriétés de barrière aux gaz, notamment à l'oxygène, à l'azote et/ou au dioxyde de carbone, et/ ou une autre couche encore pouvant conférer des propriétés de résistance chimique vis-à-vis du fluide à délivrer.

Une couche apte à conférer de bonnes propriétés de résistance mécanique peut par exemple être constituée en polyéthylène téréphtalate (PET).

Une couche apte à conférer de bonnes propriétés de barrière aux gaz peut par exemple être réalisée en Nylon, notamment Nylon MXD6, en résine éthylène-vinylalcool (ou EVOH) ou en oxyde de silicium.

Une couche apte à conférer de bonnes propriétés de résistance chimique peut par exemple, elle aussi, être constituée en polyéthylène téréphtalate ou polyéthylène naphtalate (PEN).

Ainsi, la poche de volume variable peut être réalisée en un laminé de type PET/Nylon/PET ou PET/Nylon/PEN, c'est-à-dire comportant une couche externe en polyéthylène téréphtalate, une couche intermédiaire en Nylon et une couche interne, c'est à dire une couche destinée à être en contact avec le fluide à délivrer également, en polyéthylène téréphtalate ou bien en polyéthylène naphtalate; il peut également être réalisé en un laminé de type PET/EVOH/PET ou PET/EVOH/PEN.

Un tel matériau de type PET/Nylon/PET ou PET/Nylon/PEN peut présenter l'avantage supplémentaire d'être transparent. Ainsi, si l'on veut améliorer les propriétés de transparence, il faut diminuer le pourcentage des produits conférant un effet de barrière. Par exemple, de faibles pourcentages de Nylon (3 à 8% par rapport au poids total du polymère) permettent l'obtention d'un matériau totalement transparent.

De tels laminés peuvent être réalisés par mise en oeuvre de techniques de co-extrusion ou de co-injection à l'aide de technologies telles que celles développées par la Société KORTEC Inc. Ipswich, MA01938, USA.

Il est également envisageable de déposer une couche, par exemple d'oxyde de silicium, par déposition de vapeur. La couche ainsi déposée peut être extrêmement fine, de quelques microns d'épaisseur seulement. La technologie à mettre en oeuvre est, par exemple, celle développée par la Société SIG Corpoplast Inc., sous l'appellation PLASMAX.

Une telle couche peut être déposée sur un matériau monocouche classique ou bien sur un laminé obtenu par co-extrusion ou par co-injection.

Selon un mode de réalisation particulier, ladite extrémité ouverte est dotée d'un collier de renforcement interne ou externe qui lui confère ses propriétés de stabilité dimensionnelle et qui est réalisé en un matériau moins sensible aux variations de chaleur, par exemple en Nylon 66 ou en copolymère ou polymère Acetal ou même en métal inoxydable tel que par exemple AISI 316 dans le cas d'un collier interne. Plus précisément, une partie du matériau constitutif de l'extrémité ouverte est remplacé par ledit collier ou bien externe ou bien interne, la forme générale et la dimension de l'extrémité ouverte se trouvent donc inchangées par rapport à un mode de réalisation dépourvu de tout renforcement. De façon à assurer une liaison intime entre le collier de renforcement et le matériau de l'extrémité ouverte, on procède de la façon suivante.
Dans le cas d'un collier externe, celui-ci est placé autour de l'extrémité ouverte de la poche et la surface interne dudit collier correspond intimement à la paroi externe de ladite extrémité ouverte. Pour ce faire, le collier de renforcement est placé dans le moule destiné à la réalisation de la poche et le polymère est injecté dans ledit moule.
Dans le cas d'un collier interne, la forme externe dudit collier doit être complémentaire de la forme interne de ladite extrémité ouverte. Ce collier peut être fabriqué (par avance) par un processus de moulage par injection, et doit être mis en place dans le moule d'injection /étirage-soufflage (stretch blow) par un mécanisme de prise et de mise en place mécanique.

Selon un autre mode de réalisation, au moins une partie de l'extrémité ouverte de ladite poche est réalisée en un matériau différent du reste de la poche et qui est moins sensible aux variations de chaleur, par exemple en Nylon 66 ou en copolymère ou polymère Acetal et qui est compatible avec celui-ci de telle sorte que les matériaux forment un mélange intime lorsqu'ils sont extrudés. Afin de favoriser ce mélange intime, les deux matériaux constitutifs sont introduits de façon à être forcés chacun dans une direction opposée.

Selon un autre mode de réalisation, l'extrémité ouverte de ladite poche est surdimensionnée et soumise à un traitement de stabilisation thermique « heat set » dans des conditions de temps et de températures contrôlées. Dans un tel procédé, l'extrémité ouverte est surdimensionnée puis traitée thermiquement dans des conditions de temps et de températures contrôlées et éventuellement placée dans un moule de reformage. Ce moule de reformage contient des mâchoires qui sont munies de passages qui permettent la recirculation d'un fluide de refroidissement, qui peut être, par exemple, de l'eau refroidie. Ainsi, on peut gérer la température finale de l'extrémité ouverte de la poche de volume variable. L'utilisation d'un moule de reformage et le refroidissement ont pour but non seulement de ramener le matériau à la température ambiante mais également de s'assurer que l'élément qui a été chauffé prendra les formes et dimensions précises nécessaires à son usage futur.

Bien entendu, les conditions du traitement thermique, c'est-à-dire la température et le temps dépendront de la forme, de la dimension et du matériau de ladite extrémité ouverte. Le surdimensionnement dépendra aussi des conditions de traitement thermique et des propriétés finales requises.

Le traitement thermique peut être réalisé sur une partie seulement de la surface la plus externe de ladite extrémité et non sur toute son épaisseur.

Le surdimensionnement est de l'ordre de 2 à 6%, de préférence de 3 à 5%. La durée du traitement thermique est d'environ 10s à environ 60s, de préférence d'environ 20s à environ 50s et plus préférentiellement encore d'environ 30s à environ 45s. La température est comprise entre environ 120°C et 350°C, de préférence entre environ 140°C et environ 330°C et plus préférentiellement encore entre environ 150°C et environ 290°C.

Selon un mode de réalisation particulier, la poche selon l'invention est dotée d'un dispositif de délivrance du fluide qu'elle est destinée à contenir comprenant une commande permettant d'ouvrir une valve pour délivrer ledit fluide, de préférence ledit système est un dispositif de pulvérisation.

De façon avantageuse, le système de délivrance du fluide est rendu solidaire de la poche par un capot ou coupelle permettant également de rendre l'ensemble solidaire du récipient externe dans lequel la poche est destinée à être insérée.

L'invention a également pour objet un dispositif apte à délivrer des fluides sous pression, comprenant un récipient résistant à une pression intérieure élevée par l'ouverture ou goulot duquel est introduite une poche de volume variable conforme à l'invention et telle que décrite précédemment, ladite poche contenant le fluide à délivrer et étant munie d'un dispositif de délivrance dudit fluide comprenant une commande C permettant d'ouvrir une valve V pour délivrer ledit fluide, le volume intérieur du récipient compris entre sa paroi et la poche à volume variable étant rempli d'un gaz neutre sous une pression suffisante pour exercer sur ladite poche à volume variable une contrainte pneumatique suffisante pour permettre de délivrer le fluide qu'elle contient lorsque ladite valve est actionnée par ladite commande C.

La poche est de volume variable de telle sorte que vide elle puisse être débarrassée de son contenu d'air afin de pouvoir être introduite par l'ouverture ou goulot du récipient externe et qu'elle puisse se dilater sous l'effet du fluide destiné à la remplir. De façon particulièrement avantageuse, la poche interne est dotée de plis longitudinaux comme décrit dans les demandes de brevet au nom de la Demanderesse mentionnées ci-dessus. Pour la fabrication de ladite poche, on peut avoir recours aux procédés décrits dans les quatre brevets américains identifiés plus haut et plus particulièrement dans le brevet n° 4 387 833, de la colonne 3, lignes 63 à la colonne 4, ligne 16.

Selon un mode de réalisation avantageux, ledit récipient externe ne comporte qu'une seule ouverture qui est celle par laquelle est insérée la poche de volume variable. Cette ouverture doit également permettre le remplissage dudit récipient en un gaz neutre sous pression comme ceci est décrit dans les demandes de brevet au nom de la demanderesse mentionnées ci-dessus.

Le récipient externe du dispositif de l'invention est destiné à contenir un gaz sous pression, il doit donc être capable de résister à une pression supérieure à 5 bars, de préférence supérieure à 8 bars, plus préférentiellement supérieure à 12 bars et peut même être capable de supporter des pressions supérieures à 20 bars.

Le récipient externe peut être réalisé en le même matériau que celui de la poche. Cependant, compte tenu du fait que ce matériau constitutif n'est pas en contact avec le fluide à délivrer, il est tout à fait possible d'utiliser un laminé bi-couche, par exemple en polyéthylène téréphtalate et Nylon.

Un tel matériau dépourvu de couche interne conférant une résistance chimique pourra également être mis en oeuvre pour la réalisation de ladite poche dans la mesure où le fluide à délivrer est chimiquement compatible avec les autres couches.

Selon un mode de réalisation particulier, le récipient de volume variable contenant le fluide à délivrer et le récipient extérieur résistant à la pression sont réalisés en matériaux transparents, de telle sorte que l'utilisateur voit le fluide et peut connaître à tout moment l'état de remplissage du dispositif. De même, toute altération du fluide qui conduirait à un changement de l'aspect de celui-ci (coloration, déphasage, etc.) peut être détectée par l'utilisateur.

L'invention porte également dur le procédé de remplissage d'un dispositif conforme à l'invention suivant lequel, on introduit par l'ouverture du récipient externe la poche dotée du dispositif de délivrance du fluide, on introduit le gaz sous pression par un espace prévu entre la paroi du récipient et la poche, on fixe de façon étanche les deux éléments entre eux par exemple par sertissage, vissage ou soudure, puis, en forçant la valve, on remplit la poche interne avec le fluide.

L'invention vise encore d'autres dispositions qui s'utilisent de préférence en même temps et dont il sera plus explicitement question ci-après.

Et elle sera encore mieux comprise à l'aide du complément de description de certains modes de réalisation non limitatifs, illustrés par les dessins, dans lesquels :
- les figure 1a à 1c sont une vue en coupe longitudinale d'un mode de réalisation de la poche conforme à l'invention dans ses différentes conformations volumiques,
- les figures 2a et 2b représentent chacune une vue en coupe à plus grande échelle d'un mode de réalisation de l'extrémité ouverte de ladite poche, l'une avec une forme de fixation à vis et l'autre avec une bague de fixation externe pour soudure,
- la figure 3 est une coupe longitudinale de ladite poche dotée d'un dispositif de pulvérisation,
- la figure 4 est une coupe de l'extrémité ouverte de la poche selon l'invention comportant un collier externe,
- la figure 5 est une coupe de l'extrémité ouverte de la poche selon l'invention comportant un collier de renfort interne,
- la figure 6 est une coupe de l'extrémité ouverte de la poche selon l'invention réalisée en un matériau différent de celui de la poche,
- la figure 7 est une coupe d'un dispositif conforme à l'invention rempli.

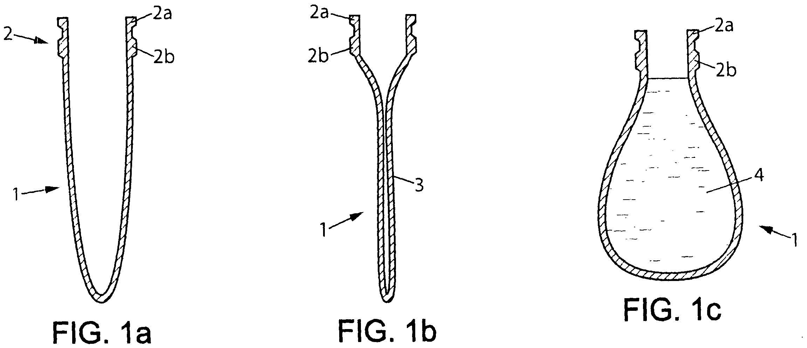

Comme montré sur les figures 1a à 1b, le récipient 1 conforme à l'invention est de volume variable et se présente sous la forme d'une poche comportant une extrémité ouverte 2 dotée sur sa partie externe de protubérances 2a et 2b. Sur la figure 1a, la poche est représentée sous sa forme libre, c'est-à-dire lorsqu'elle n'est soumise à aucune contrainte. Sur la figure 1b, la poche est représentée après avoir été vidée de son air ou bien par aspiration ou par pression mécanique sur sa paroi 3. Sur la figure 1c est représentée une poche remplie de liquide 4, le volume de la poche est alors augmenté par rapport au volume de la poche soumise à aucune contrainte.

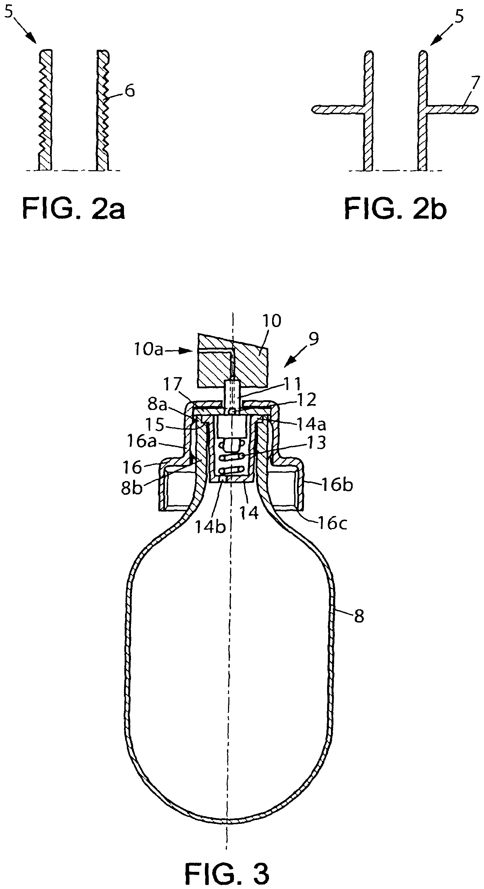

Sur les figures 2a et 2b sont représentés deux modes de réalisations différents de l'extrémité ouverte 5 de la poche selon l'invention. Sur la figure 2a, l'extrémité ouverte 5 est dotée sur sa partie externe d'un pas de vis 6. Sur la figure 2b, ladite extrémité ouverte est dotée d'une bague radiale 7.

Sur la figure 3 est représenté un récipient 8 de volume variable doté d'un système de pulvérisation 9 comprenant un bouton poussoir 10 actionnant une tige creuse de commande 11 percée d'un trou 12 permettant la communication entre l'intérieur de la poche et la cavité de la tige de commande, ledit bouton poussoir étant percé d'un canal 10a disposé dans le prolongement de la cavité de la tige creuse de commande 11, ladite tige de commande 11 faisant pression sur un ressort 13 maintenu dans une cuvette de ressort 14, le bord libre 14a de ladite cuvette prenant appui sur un épaulement 15 ménagé sur la face interne du bord libre 8a de la poche, l'ensemble cuvette-ressort-tige de commande étant maintenu par un capot 16 vissé sur l'extrémité ouverte 8b de ladite poche. La cuvette 14 est dotée d'un trou 14b qui permet le passage du fluide à délivrer depuis la poche de volume variable vers le trou 12 de la tige 11 de commande. La paroi externe de l'extrémité ouverte 8b est dotée d'un pas de vis complémentaire du pas de vis ménagé sur la paroi latérale 16a interne du capot 16. Un joint plat 17 assurant l'étanchéité entre la tige de commande 11 et le bord libre 8a de la poche 8 et également entre la poche 8 et le capot 16. Le capot 16 est doté d'une extension 16b de diamètre supérieur au diamètre de la partie 16a coopérant avec l'extrémité ouverte 8b de la poche et dont la face interne est dotée d'un filetage 16c. Ce filetage est destiné à coopérer avec le filetage d'un récipient externe dans lequel ladite poche 8 est destinée à être insérée.

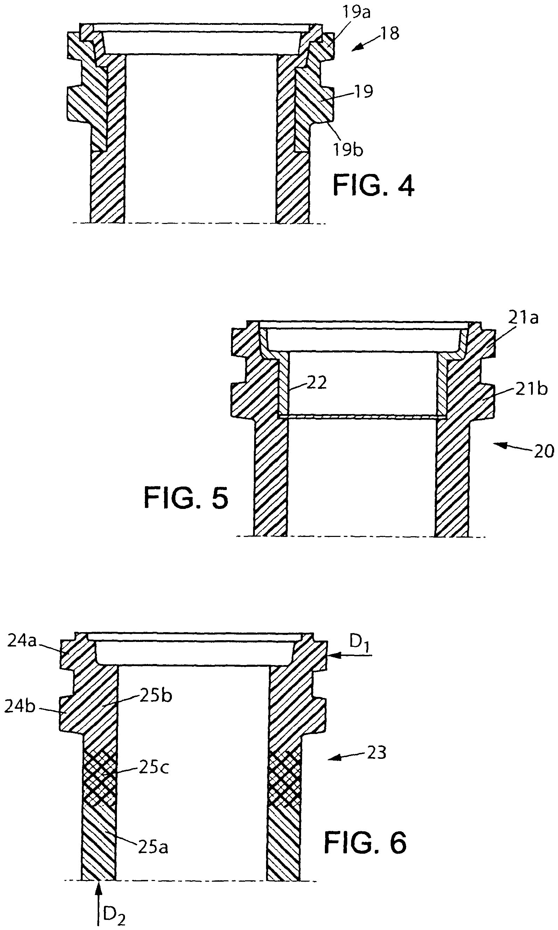

La figure 4 représente l'extrémité ouverte 18 de la poche selon l'invention qui est dotée sur sa paroi externe d'un collier 19 de renforcement comportant sur sa périphérie externe deux protubérances 19a et 19b.

La figure 5 représente l'extrémité ouverte 20 de la poche selon l'invention qui est dotée sur sa paroi externe deux protubérances 21a et 21b et dont la paroi interne est complémentaire de la forme externe d'un collier 22 de renforcement.

La figure 6 représente l'extrémité ouverte 23 d'un autre mode de réalisation de la poche conforme à l'invention qui comprend une paroi externe dotée de deux protubérances 24a et 24b, ladite paroi étant réalisée en deux matériaux distincts, le matériau de la poche dans la partie inférieure 25a, un matériau moins sensible aux variations thermiques dans la partie supérieure 25b (c'est-à-dire la plus proche de l'extrémité libre) et un matériau mixte dans la zone intermédiaire 25c. Sur cette figure sont représentées par les flèches D1 et D2 le sens d'injection des matériaux constitutifs. Ainsi, le matériau de la poche est injecté du fond de la poche vers son extrémité ouverte et le matériau moins sensible aux variations thermiques est injecté dans la protubérance 24a perpendiculairement à D2 et en imposant une poussée inverse par rapport à celle du matériau de la poche. Ainsi les deux matériaux se mélangent au niveau de la zone 25c.

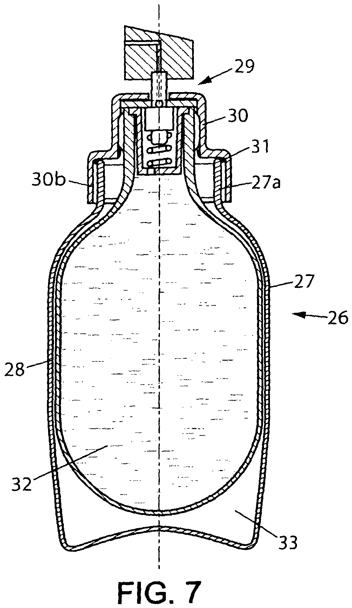

Sur la figure 7 est représenté un dispositif conforme à l'invention désigné dans son ensemble par 26. Ce dispositif comporte un récipient externe 27 de forme générale cylindrique ne comportant qu'une seule ouverture en sa partie supérieure 27a. La paroi externe de la partie 27a comporte un pas de vis. Par l'ouverture dudit récipient 27 été introduite une poche 28 dotée d'un dispositif 29 de pulvérisation tel que représenté sur la figure 3. Le récipient externe est vissé sur le capot du dispositif de pulvérisation 29 dont la paroi interne de l'extension 30b est dotée d'un pas de vis correspondant. Un joint torique 31 assure l'étanchéité entre le bord libre du récipient externe 27 et le capot 30. Ladite poche 28 est remplie de liquide 32 et l'espace 33 compris entre la paroi intérieure du récipient externe 27 et ladite poche 28 est rempli de gaz sous pression.

En suite de quoi, et quel que soit le mode de réalisation adopté, on dispose d'un dispositif du genre en question dont les caractéristiques résultent suffisamment de ce qui précède pour qu'il soit inutile d'insister à ce sujet, ce dispositif présentant, par rapport à ceux qui existent déjà, de nombreux avantages dont notamment celui d'une grande fiabilité, celui d'une grande robustesse, celui d'un prix de revient compétitif et celui d'une parfaite étanchéité même dans des conditions extrêmes d'utilisation et de conservation.

## Revendications

1. Poche de volume variable (1 ;8 ;28) apte à contenir des fluides et destinée à être introduite dans un récipient (27) par le goulot de celui-ci, constituée d'un sac fermé à l'une de ses extrémités et ouverte à l'autre extrémité, l'extrémité ouverte (2 ;5 ;18 ;20 ;23) étant stable dimensionnellement sur des plages de températures allant de -30 à 55°C, et le facteur de dilatation de ladite extrémité étant divisé par 1,5, au moins une partie de l'extrémité ouverte (2 ;5 ;18 ;20 ;23) étant réalisée en un matériau moins sensible aux variations thermiques que le matériau constitutif de la poche.

2. Poche (1 ;8 ;28) selon la revendication 1, **caractérisée par le fait que** la paroi externe de l'extrémité ouverte est dotée d'un pas de vis (6).

3. Poche (1 ;8 ;28) selon la revendication 1, **caractérisée par le fait que** la paroi externe de l'extrémité ouverte comporte au moins une protubérance radiale (7) destinée à un sertissage.

4. Poche (1 ;8 ;28) selon la revendication 1, **caractérisée par le fait que** la paroi externe de l'extrémité ouverte présente au moins une bague latérale destinée à une soudure.

5. Poche (1 ; 8 ;28) selon l'une quelconque des revendications 1 à 4, **caractérisée par le fait que** l'extrémité ouverte est constituée en partie d'un collier de renforcement interne (22) ou externe (19).

6. Poche (1 ;8 ;28) selon la revendication 5, **caractérisée par le fait que** le collier de renforcement (19 ;22) est réalisé en un matériau moins sensible aux variations thermiques que le matériau constitutif de la poche (1 ;8 ;28).

7. Poche (1 ;8 ;28) selon l'une quelconque des revendications 1 à 6, **caractérisée par le fait qu'**elle est dotée d'un dispositif de délivrance du fluide qu'elle est destinée à contenir comprenant une commande C permettant d'ouvrir une valve V pour délivrer ledit fluide, de préférence ledit système est un dispositif de pulvérisation.

8. Dispositif apte à délivrer des fluides sous pression, comprenant un récipient 27 résistant à une pression intérieure élevée par l'ouverture ou goulot duquel est introduite une poche de volume variable telle que définie à l'une quelconque des revendications 1 à 6, ladite poche contenant le fluide à délivrer et étant munie d'un dispositif de délivrance dudit fluide comprenant une commande C permettant d'ouvrir une valve V pour délivrer ledit fluide, le volume intérieur du récipient compris entre sa paroi et la poche à volume variable étant rempli d'un gaz neutre sous une pression suffisante pour exercer sur ladite poche à volume variable une contrainte pneumatique suffisante pour permettre de délivrer le fluide qu'elle contient lorsque ladite valve est actionnée par ladite commande C.

9. Dispositif selon la revendication 8, **caractérisé par le fait que** la poche (1 ;8 ;28) et le récipient externe (27) sont réalisés en des matériaux transparents.

10. Procédé de remplissage d'un dispositif selon l'une quelconque des revendications 1 à 9, suivant lequel, on introduit par l'ouverture du récipient externe(27) la poche (1 ;8 ;28) dotée du dispositif de délivrance du fluide, on introduit un gaz sous pression par un espace prévu entre la paroi du récipient (27) et la poche (1 ;8 ;28), on fixe de façon étanche les deux éléments entre eux par exemple par sertissage, vissage ou soudure, puis, en forçant la valve, on remplit la poche interne avec le fluide.

## Claims

1. Pouch having a variable volume (1; 8; 28) capable of containing fluids and intended to be inserted into a container (27) through the neck thereof, constituted by a bag that is closed at one end and open at the other end, the open end (2; 5; 18; 20; 23) being dimensionally stable over temperature ranges from -30 to 55°C, and the expansion factor of said end being divided by 1.5, at least a part of the open end (2; 5; 18; 20; 23) being produced from a material that is less sensitive to thermal variations than the material constituting the pouch.

2. Pouch (1; 8; 28) according to claim 1, **characterized by** the fact that the outer wall of the open end is equipped with a screw thread (6).

3. Pouch (1; 8; 28) according to claim 1, **characterized by** the fact that the outer wall of the open end includes at least one radial projection (7) intended for crimping.

4. Pouch (1; 8; 28) according to claim 1, **characterized by** the fact that the outer wall of the open end has at least one lateral ring intended for welding.

5. Pouch (1; 8; 28) according to any one of claims 1 to 4, **characterized by** the fact that the open end is partially constituted by an inner (22) or outer (19) reinforcing collar.

6. Pouch (1; 8; 28) according to claim 5, **characterized by** the fact that the reinforcing collar (19; 22) is made from a material that is less sensitive to thermal variations than the material constituting the pouch (1; 8; 28).

7. Pouch (1; 8; 28) according to any one of claims 1 to 6, **characterized by** the fact that it is equipped with a device for dispensing the fluid that it is intended to contain, comprising a control C allowing a valve V to be opened to dispense said fluid, said system preferably being a spray device.

8. Device capable of dispensing fluids under pressure, comprising a container 27 capable of withstanding a high internal pressure, through the opening or neck of which there is introduced a variable-volume pouch as defined in any one of claims 1 to 6, said pouch containing the fluid to be dispensed and being fitted with a device for dispensing said fluid, comprising a control C allowing a valve V to be opened to dispense said fluid, the internal volume of the container comprised between its wall and the variable-volume pouch being filled with an inert gas under sufficient pressure to exert on said variable-volume pouch a pneumatic stress sufficient to allow the fluid which it contains to be dispensed when said valve is actuated by said control C.

9. Device according to claim 8, **characterized by** the fact that the pouch (1; 8; 28) and the outer container (27) are made from transparent materials.

10. Method for filling a device according to any one of claims 1 to 9, according to which there is introduced through the opening of the outer container (27) the pouch (1; 8; 28) equipped with the fluid dispensing device, a pressurized gas is introduced through a space provided between the wall of the container (27) and the pouch (1; 8; 28), the two components are sealed together by for example crimping, screwing or welding, then the inner pouch is filled with the fluid by forcing the valve.

## Patentansprüche

1. Tasche mit veränderlichem Volumen (1; 8; 28), welche dazu geeignet ist, Flüssigkeiten zu enthalten und dazu bestimmt ist, in einen Behälter (27) durch dessen Flaschenhalsöffnung aufgenommen zu werden, bestehend aus einem Beutel, welcher an einem seiner Enden geschlossen und am anderen Ende offen ist, wobei die Abmessungen des offenen Endes (2; 5; 18; 20; 23) in Temperaturbereichen von -30 bis 55 °C stabil sind, wobei der Ausdehnungsfaktor des Endes durch 1,5 geteilt ist und wobei wenigstens ein Teil des offenen Endes (2; 5; 18; 20; 23) in einem Material ausgeführt ist, welches unempfindlicher gegen Temperaturschwankungen ist als das Grundmaterial der Tasche.

2. Tasche (1; 8; 28) nach Anspruch 1, **dadurch gekennzeichnet, dass** die Außenwand des offenen Endes mit einer Gewindesteigung (6) versehen ist.

3. Tasche (1; 8; 28) nach Anspruch 1, **dadurch gekennzeichnet, dass** die Außenwand des offenen Endes wenigstens einen für einen Bördelvorgang bestimmten radialen Überstand (7) umfasst.

4. Tasche (1; 8; 28) nach Anspruch 1, **dadurch gekennzeichnet, dass** die Außenwand des offenen Endes wenigstens einen für einen Schweißvorgang bestimmten seitlichen Ring aufweist.

5. Tasche (1; 8; 28) nach einem der Ansprüche 1 bis 4, **dadurch gekennzeichnet, dass** das offene Ende teilweise aus einer inneren (22) oder äußeren (19) Verstärkungsschelle gebildet ist.

6. Tasche (1; 8; 28) nach Anspruch 5, **dadurch gekennzeichnet, dass** die Verstärkungsschelle (19; 22) in einem Material ausgeführt ist, welches unempfindlicher gegen Temperaturschwankungen ist als das Grundmaterial der Tasche (1; 8; 28).

7. Tasche (1; 8; 28) nach einem der Ansprüche 1 bis 6, **dadurch gekennzeichnet, dass** diese mit einer Zuführvorrichtung für die Flüssigkeit versehen ist, welche sie enthalten soll, umfassend eine Steuerung C, welche es ermöglicht, ein Ventil V zum Zuführen der Flüssigkeit zu öffnen, wobei das System vorzugsweise eine Zerstäubungsvorrichtung ist.

8. Vorrichtung, welche dazu geeignet ist, unter Druck stehende Flüssigkeiten abzugeben, umfassend einen Behälter 27, der einem erhöhten Innendruck widersteht und durch dessen Öffnung oder Flaschenhals eine Tasche mit veränderlichem Volumen, wie in einem der Ansprüche 1 bis 6 beschrieben, eingeführt ist, wobei die Tasche die abzugebende Flüssigkeit enthält und mit einer Abgabevorrichtung für die Flüssigkeit versehen ist, umfassend eine Steuerung C, welche es ermöglicht, ein Ventil V zum Abgeben der Flüssigkeit zu öffnen, wobei das Innenvolumen des Behälters zwischen seiner Wand und der Tasche mit veränderlichem Volumen mit einem neutralen Gas gefüllt ist, welches unter einem ausreichenden Druck steht, um auf die Tasche mit veränderlichem Volumen einen ausreichenden pneumatischen Zwang auszuüben, um zu ermöglichen , dass die darin enthaltene Flüssigkeit abgegeben wird, wenn das Ventil durch die Steuerung C aktiviert ist.

9. Vorrichtung nach Anspruch 8, **dadurch gekennzeichnet, dass** die Tasche (1; 8; 28) und der äußere Behälter (27) aus transparenten Materialien ausgeführt sind.

10. Füllverfahren für eine Vorrichtung nach einem der Ansprüche 1 bis 9, wobei durch die Öffnung des äußeren Behälters (27) die mit der Flüssigkeitsabgabevorrichtung für die Flüssigkeit versehene Tasche (1; 8; 28) eingeführt wird, ein unter Druck stehendes Gas durch einen zwischen der Wand des Behälters (27) und der Tasche (1; 8; 28) vorgesehenen Raum eingeführt wird, die zwei Elemente beispielsweise mittels Börderln, Verschrauben oder Schweißen untereinander dicht befestigt werden und dann durch Kraftausübung auf das Ventil die innere Tasche mit der Flüssigkeit gefüllt wird.
